(19) Europäisches Patentamt
European Patent Office
Office européen des brevets

(11) EP 0 689 052 B1

(12) EUROPEAN PATENT SPECIFICATION

(45) Date of publication and mention of the grant of the patent:
**26.09.2001 Bulletin 2001/39**

(51) Int Cl.⁷: **G01N 33/96**, G01N 33/74

(21) Application number: **95610040.8**

(22) Date of filing: **23.06.1995**

# (54) Insulin standard solution

Insulinstandardlösung

Solution standard d'insuline

(84) Designated Contracting States:
**DE FR GB IT**

(30) Priority: **24.06.1994 JP 14351194**

(43) Date of publication of application:
**27.12.1995 Bulletin 1995/52**

(73) Proprietor: **Sysmex Corporation**
**Kobe-shi, Hyogo 651-0073 (JP)**

(72) Inventor: **Fukuda, Shigehiro,**
**c/o Toa Medical Elec. Co., Ltd.**
**Kobe-shi, Hyogo-ken (JP)**

(74) Representative:
**Plougmann, Vingtoft & Partners A/S**
**Sankt Annae Plads 11,**
**P.O. Box 3007**
**1021 Copenhagen K (DK)**

(56) References cited:
**EP-A- 0 046 523**
**EP-A- 0 382 875**
**GB-A- 1 527 605**
**US-A- 3 975 511**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

BACKGROUND OF THE INVENTION

1. Field of the Invention

The present invention relates to an insulin standard solution (insulin calibrator) which is used for measuring insulin concentration contained in a test sample by utilizing an immunoreaction.

2. Description of the Related Art

In a test where an antigen or antibody is quantatively determined by utilizing an immunoreaction, a calibration curve is previously prepared by measuring specimens (calibrator) containing prescribed amounts of an antigen (for example, bioactive such as hormone) or antibody, a test sample is measured and the concentration of antigen or antibody contained in the test sample is calculated by using the calibration curve. The calibrator can be stabilized by various methods. For example, an conventional insulin calibrator is a freeze-dried product or a product having high concentration. It is used by dissolving in water or diluting to an appropriate concentration.

In addition, Japanese Patent Publication No. Sho 60 (1985)-30651 describes that a surfactant is added to an insulin solution which is used as a nasal liquid formulation to improve storage stability.

In general, calibrators are required to have storage stability for a long period and also to show the same behavior to a immunoreaction of insulin as a test sample taken from a patient. Suppose the concentration of insulin is the same, a measured value obtained from the test sample must show the same as in the calibration. In other words, a substance added to the insulin standard solution as a stabilizer should not adversely affect a reaction system for measuring insulin concentration.

In accordance with experiments conducted by the inventors of the present invention, it is noted that when various surfactants are added to the insulin standard solution, the immunoaggregation reaction is enhanced/inhibited compared with the case where the surfactant is not added, thereby showing a high/low insulin concentration in appearance which is different from a real insulin concentration. Such standard insulin solution is quite unsuitable.

Moreover, when dissolving the freeze-dried insulin or diluting the insulin standard solution having high concentration, there is a problem of causing an experimental error at dissolving or diluting operation. Further, in case of the freeze-dried insulin, stability after dissolving is not good.

SUMMARY OF THE INVENTION

The present invention provides an insulin standard solution for immunoassay, said solution comprising an amide derivative of bile acid as a stabilizer having the formula (I):

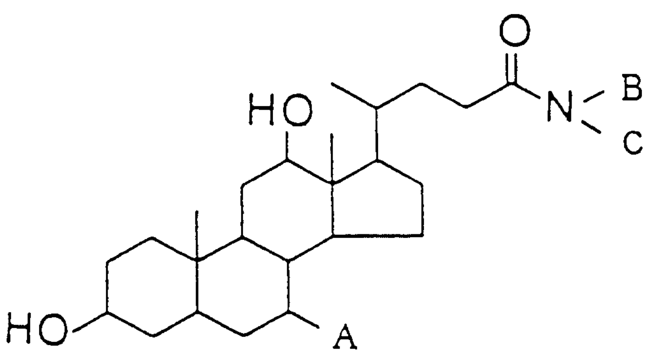

(I)

wherein A is a hydrogen atom or a hydroxyl group, B has the formula:

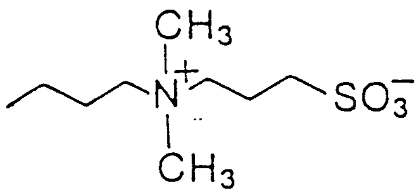
,
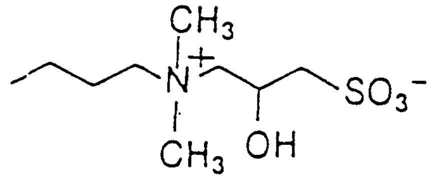

or

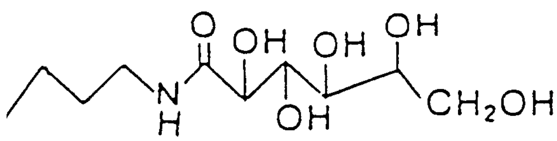

and C is a hydrogen atom or a group having the formula:

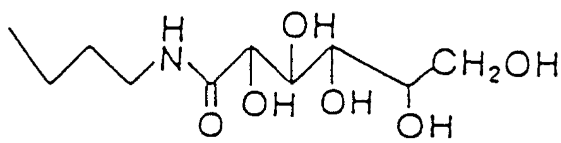

Also the present invention provides an insulin standard solution showing the same behavior as a test sample taken from a patient, while maintaining storage stability for a long period.

BRIEF DESCRIPTION OF THE DRAWINGS

Fig. 1 shows a relationship between storage period of an insulin standard solution of the present invention and insulin persistence.

DETAILED DESCRIPTION OF THE INVENTION

The insulin standard solution of the present invention comprises an insulin, a buffer solution and a compound having the formula (I) as a stabilizing agent, whereby the insulin standard solution can maintain its storage stability for a long period and does not affect immunoaggregation even if the insulin standard solution has low concentration.

The insulin may be an insulin generally used for any insulin used for treatment for diabetes and research thereof, preferably a regular one without including various additions. Examples of the insulin include a formulation described in Pharmacopoeia of Japan derived from human, horse, swine, bovine, and the like, such as a recombinant human insulin (Eli Lilly and Co.: Humalin), amino acid converting human insulin (Novo-Nordisk A/S: Actrapid Human), but are not specifically limited thereto. Preferred concentration of insulin contained in the insulin standard solution ranges from 1 to 1,000 μU/ml.

The buffer solution may be any buffer solution generally used for storing hormones such as insulin and an antigen or antibody. Examples of the buffer solutions include TRIS buffer solution, HEPES buffer solution and phosphate buffer solution.

Specific examples of the amide derivatives of bile acid having the formula (I) include 3-[(3-cholamidopropyl) dimethylammonio]-1-propanesulfonate (CHAPS), 3-[(3-cholamidopropyl)dimethylammonio]-2-hydroxy-1-propanesulfonate (CHAPSO), N,N-bis(3-D-gluconamidopropyl)- cholamide (BIG-CHAP) and N,N-bis(3-D-gluconamidopropyl)deoxycholamide (deoxy-BIGCHAP), among which CHAPS is most preferable. In EP-A-0 382 875, CHAPS has been used as a physical stabilizer for fibroblast growth factor (FGF). The amide derivative of bile acid is required to be added at a concentration which does not affect the immunoreaction of insulin standard solution, preferably ranging from 0.01 to 1.0 W/V% to the standard insulin solution, more preferably 0.1 to 0.4 W/V%. If the concentration is too low, the storage stability is not maintained for a long period, while when the concentration is too high, immunoaggregation may be affected, thereby preventing from measuring correct insulin concentration.

Preferably, the insulin standard solution has a pH of 5.0 to 9.0, more preferably, around 7.0. The pH value is adjusted by using a known buffer agent.

Further, in order to inhibit proliferation of bacteria, it would be convenient to add an antibacterial substance such as sodium azide to the insulin standard solution at a concentration which does not adversely affect the immunoreaction of insulin.

The insulin standard solution of the present invention can be used not only for immunoaggregation, but also for other known immunoassay such as radioimunoassays (RIA) and enzyme immunoassay (EIA).

Examples of the insulin standard solution of the present invention are described as follows.

Example 1

Three kinds of 10 mM PBS buffer solutions (pH 7.0) containing 17.0 µU/ml (L), 69.0 µU/ml (M) or 175.0 µU/ml (H) of recombinant human insulin respectively were prepared.

To each of these PBS buffer solutions was added 0.1 W/V% or 1.0 W/V% CHAPS to prepare an insulin standard solution, and insulin concentration in the insulin standard solution was measured as follows. First, 10 µl of the insulin standard solution, 10 µl of an anti-insulin antibody sensitized latex suspension and 80 µl of an aqueous solution containing 10 mM Tris buffer solution(pH 6.0) were mixed and reacted for 15 minutes at 45°C. Then, the insulin concentration was measured by using immunoaggregation measuring device PAMIA-10 (manufactured by TOA Medical Electronics., Co., Ltd.). The results are shown in Table 1.

Separately, a control was prepared by mixing 10µl of 10 mM PBS buffer solution (pH 7.0) containing only recombinant human insulin, 10 µl of an anti-insulin antibody sensitized latex suspension and 80 µl of an aqueous solution containing 10 mM Tris buffer solution(pH 6.0). Then, the insulin concentration was measured in the same manner as described above.

Further, as comparative examples, Triton X-100, NP-40 (manufactured by Nikko Chemicals, 50% aqueous solution containing polyoxyethylene(40)nonylphenylether) and sodium dodecylsulfate were used respectively instead of using CHAPS to prepare 0.1 W/V% and 1.0 W/V% insulin standard solutions. Then, the insulin concentration was measured in the same manner as described above.

Table 1

(Unit:µU/ml)

| Name | Concentration (%) | Recombinant human insulin | | |
|---|---|---|---|---|
| | | L(17.0) | M(69.0) | H(175.0) |
| Control | 0 | 17.35 | 69.00 | 174.92 |
| CHAPS (The present invention) | 0.1 | 18.09 | 70.61 | 180.87 |
| | 1.0 | 18.91 | 71.56 | 179.98 |
| Triton X-100 (Comparative Ex.) | 0.1 | 35.33 | 115.16 | 259.04 |
| | 1.0 | 14.96 | 36.49 | 84.24 |
| NP-40 (Comparative Ex.) | 0.1 | 43.10 | 139.54 | 305.22 |
| | 1.0 | 17.45 | 45.42 | 101.86 |
| Sodium dodecylsulfate (Comparative Ex.) | 0.1 | 17.75 | 71.04 | 179.30 |
| | 1.0 | 9.33 | 39.71 | 119.92 |

As is clearly seen from Table 1, when 10mM PBS buffer solution containing insulin only (control) or insulin standard solution containing CHAPS (the present invention) is used, measured value of insulin concentration corresponds to the known value as used well. Especially, it was found that CHAPS did not affect insulin measurement.

In contrast, when Triton X-100 or NP-40 was used at 0.1 or 1.0 W/V%, insulin concentration in appearance

was high at 0.1 W/V%, while it was low at 1.0 W/V%. In addition, experimental errors caused by dilution were great and the calibration curve itself was changed. In case of sodium dodecylsulfate, the insulin concentration in appearance was not affected at the use of 0.1 W/V%, but the concentration was low at the use of 1.0 W/V%.

Example 2

To 10 mM PBS buffer solution containing 91.22 μU/ml (initial concentration) of recombinant human insulin was added 0.1 W/V% CHAPS to prepare an insulin standard solution. A control was prepared in the same manner expect that CHAPS was not added. The resulted solution was stored at 37°C for a predetermined period and the insulin concentration was measured in the same manner as in Example 1. The results are shown in Figure 1.
As is clearly seen from Figure 1, stability of the insulin standard solution containing 0.1 W/V% CHAPS was good compared with the solution without containing CHAPS. The insulin persistence shown in Fig. 1 denotes a value obtained by

insulin concentration after predetermined period / insulin initial concentration x 100(%).

According to the insulin standard solution of the present invention, it can maintain the storage stability for a long period even if insulin concentration is low, and it can show the same behavior to the immunoreaction of insulin as a test sample taken from a patient. As a result, the insulin concentration of the test sample taken from a patient can be correctly measured.
Further, the present invention can simplify a procedure in use by omitting a step for dissolving a conventional freeze-dried product which is required in use because the insulin standard solution of the present invention is provided in the form of an aqueous solution. Moreover, experimental errors due to the dissolving step can be eliminated.

**Claims**

1. An insulin standard solution for immunoassay, said solution comprising an amide derivative of bile acid as a stabilizer having the formula (I):

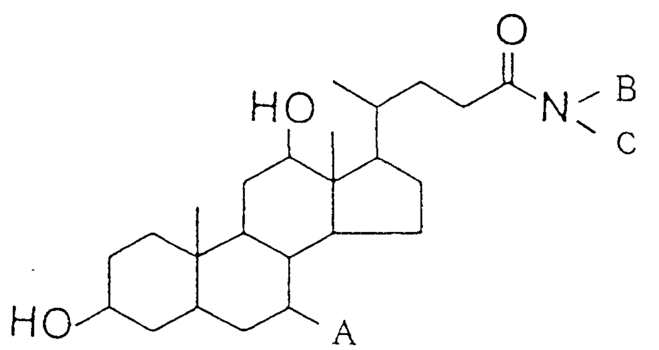

(I)

wherein A is a hydrogen atom or a hydroxyl group, B is a group having the formula:

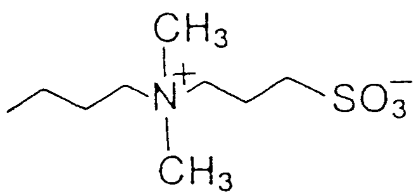

,

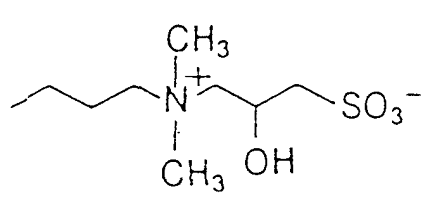

or

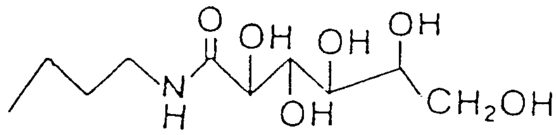

and C is a hydrogen atom or a group having the formula:

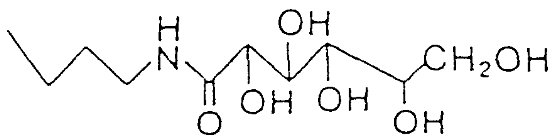

2. An insulin standard solution according to Claim 1, in which the amide derivative of bile acid having the formula (I) is 3-[(3-cholamidopropyl)dimethylammonio]-1-propanesulfonate.

3. An insulin standard solution according to Claim 1 or Claim 2, in which the amide derivative of bile acid having the formula (I) is contained at concentration of 0.1 W/V% to 1.0 W/V%.

4. An insulin standard solution according to any one of Claim 1, 2 or 3, in which insulin is contained at concentration of 1 μU/ml to 1,000 μU/ml.

**Patentansprüche**

1. Insulin-Standardlösung für einen Immunoassay, die Lösung umfassend ein Amid-Derivat von Gallensäure als ein Stabilisator mit der Formel (I):

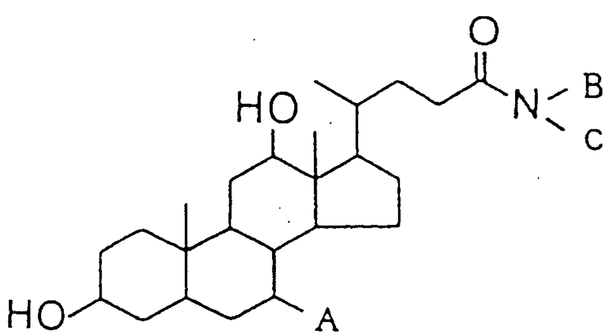

(I)

worin A ein Wasserstoffatom oder eine Hydroxyl-Gruppe ist, B ist eine Gruppe mit der Formel:

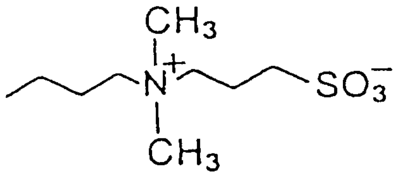

,

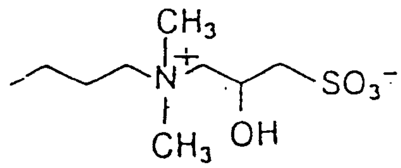

oder

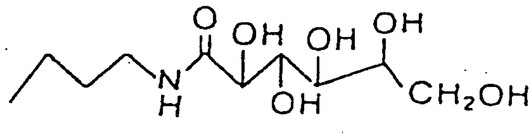

und C ist ein Wasserstoffatom oder eine Gruppe mit der Formel:

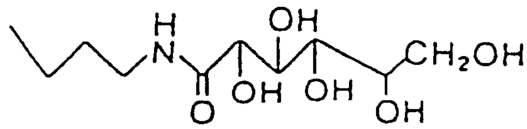

**2.** Insulin-Standardlösung gemäß Anspruch 1, in der das Amid-Derivat von Gallensäure mit der Formel (I) 3-[(3-Cholamidopropyl)dimethylammonio]-1-propansulfonat ist.

**3.** Insulin-Standardlösung gemäß Anspruch 1 oder 2, in der das Amid-Derivat von Gallensäure mit der Formel (I) in einer Konzentration von 0,1 G/V % bis 1,0 G/V % enthalten ist.

**4.** Insulin-Standardlösung gemäß einem der Ansprüche 1, 2 oder 3, in der Insulin in einer Konzentration von 1 µU/ml bis 1 000 µU/ml enthalten ist.

**Revendications**

**1.** Solution standard d'insuline pour dosage immunologique, ladite solution comprenant en tant que stabilisant, un dérivé amide d'acide biliaire de formule (I)

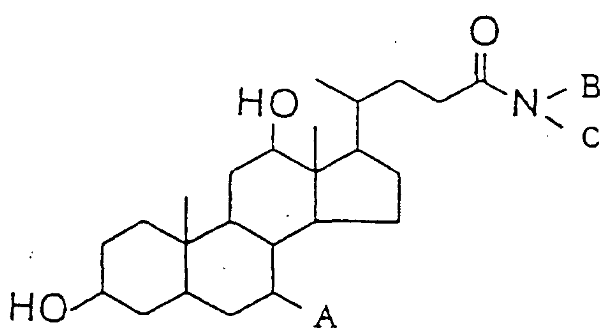

(I)

dans laquelle A représente un atome d'hydrogène ou un groupe hydroxyle, et B a la formule:

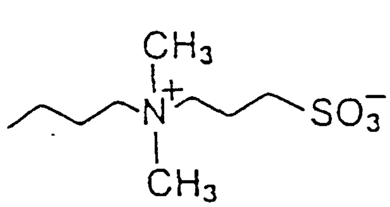

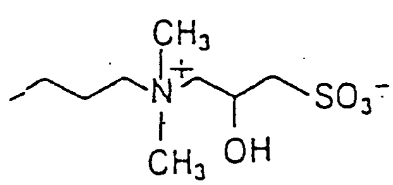

ou

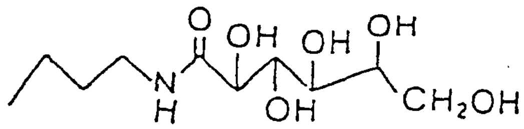

et C représente un atome d'hydrogène ou un groupe ayant la formule :

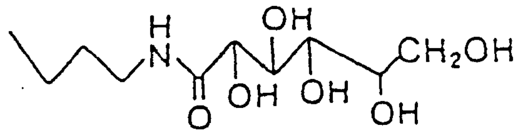

2. Solution standard d'insuline selon la revendication 1, dans laquelle le dérivé amide d'acide biliaire ayant la formule (I) est le 3-[(3-cholamidopropyl)diméthylammonio]-1-propanesulfonate.

3. Solution standard d'insuline selon la revendication 1, dans laquelle le dérivé amide d'acide biliaire ayant la formule (I) est contenu à une concentration comprise entre 0,1 % p/v et 1,0 % p/v.

4. Solution standard d'insuline selon l'une quelconque des revendications 1, 2 ou 3, dans laquelle l'insuline est contenue à une concentration comprise entre 1 µU/ml et 1.000 µU/ml.

Fig. 1

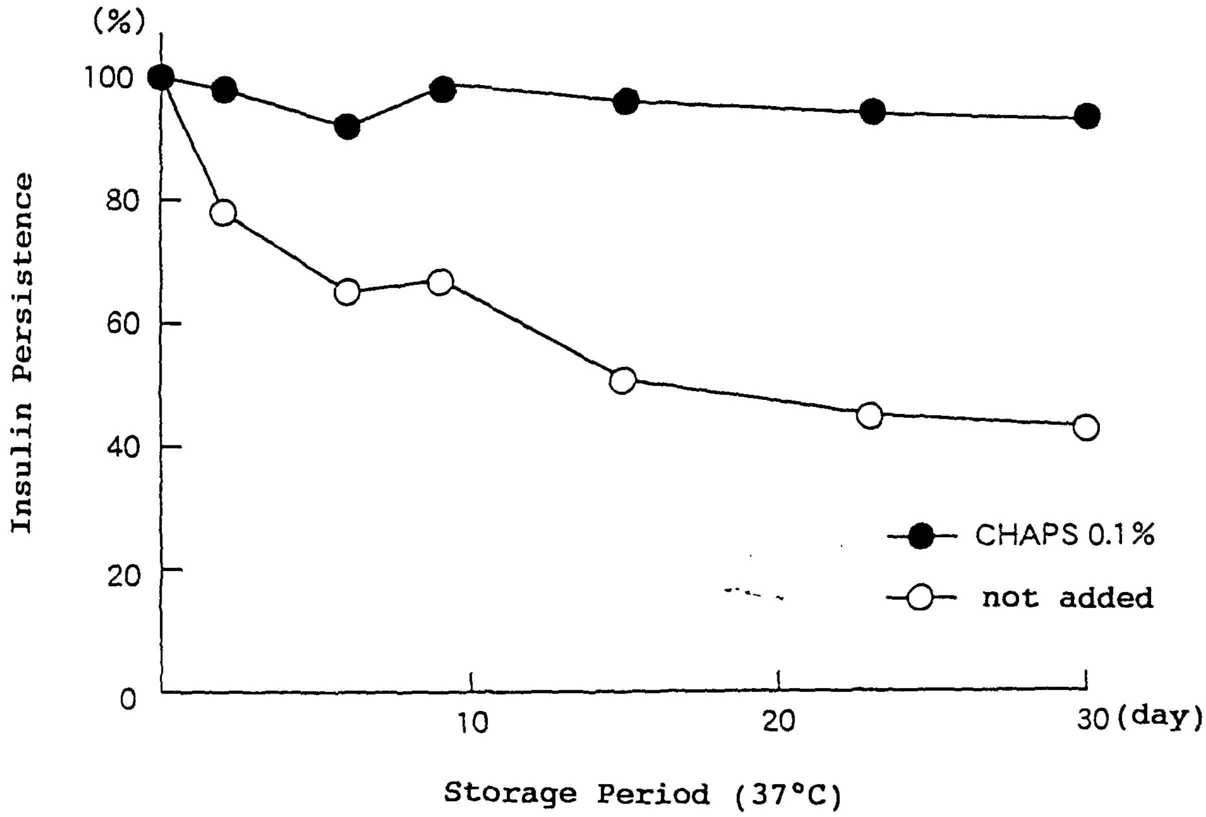
(%)
100
80
60
40
20
0
Insulin Persistence
10
20
30 (day)
Storage Period (37°C)
CHAPS 0.1%
not added